# EUROPEAN PATENT APPLICATION

(11) **EP 4 321 971 A1**
(43) Date of publication of application: **14.02.2024**
(21) Application number: 22189896.8
(22) Date of filing: 11.08.2022
(51) Int. Cl.: G06F 3/01, A61B 5/11

(54) **CONTROLLING VIRTUAL CONTENT**

(71) Applicant: Koninklijke Philips N.V., 5656 AG Eindhoven (NL)
(72) Inventor: KUHLMANN, Daan, Eindhoven (NL); TALGORN, Elise Claude Valentine, Eindhoven (NL); GEURTS, Lucas Jacobus Franciscus, 5656AG Eindhoven (NL); BUIL, Vincentius Paulus, 5656AG Eindhoven (NL); HENDRIKS, Monique, 5656AG Eindhoven (NL); LEUFKENS, Timmy Robertus Maria, 5656AG Eindhoven (NL)
(74) Representative: Philips Intellectual Property & Standards

(57) **Abstract**

Proposed concepts thus aim to provide schemes, solutions, concept, designs, methods and systems pertaining to controlling the presentation of virtual content, provided to a user by a mixed reality system. In particular, embodiments aim to obtain user posture and user activity information, and by analysing it, to determine whether at least one of the user's posture and activity needs adjusting, and if so, determining a user adjustment. During analysis of the user posture and user activity information, ergonomics data describing reference postures and activities is considered in order to determine a user adjustment. After the user adjustment has been determined, an element adjustment of the presentation of at least one element of virtual content can be determined based on the user adjustment. A control signal for implementing the element adjustment can then be generated in order to communicate with the mixed reality system or an application of the mixed reality system.

## Description

### FIELD OF THE INVENTION

This invention relates to the field of virtual content, and in particular to the field of controlling the presentation of virtual content.

### BACKGROUND OF THE INVENTION

There is increasing interest in mixed reality systems for use in a number of use-case scenarios, such as virtual offices or gaming. Mixed reality systems include presenting elements of virtual content to the user, either overlaid on the real world, i.e. augmented reality, or in a fully virtual environment, i.e. virtual reality.

As mixed reality head mounted displays become more common, so too will the problems that come along with using one. For example, repetitive strain injuries (RSI) are a well-known problem amongst PC users and a good work posture is required to avoid getting RSI. For this purpose, there are many guidelines or tools to help PC users. For mixed reality systems there are guidelines, but there are currently no tools to provide active support to help users avoid injuries.

Head mounted displays can be heavy and neck injuries can occur from daily usage. Muscles can also get sore and prolonged muscle pain can be endured by a user using a mixed reality system for too long. For instance, when a user is using the mixed reality system for more than 30 minutes, the neck may start to endure strains, but as the user is engaged in a task, they may ignore the pain or the advised guidance to take a break after 30 minutes.

Hence, there is a need for a solution to actively help the user of a mixed reality system avoid common injuries while using the system.

### SUMMARY OF THE INVENTION

The invention is defined by the claims.

According to examples in accordance with an aspect of the invention, there is provided a computer-implemented method for controlling the presentation of virtual content, provided to a user by a mixed reality system.

The method comprises: obtaining user posture and user activity information describing posture and activity of the user respectively; determining, by analyzing the obtained user posture and user activity information, and based at least partly on ergonomics data describing reference postures and activities, a user adjustment of at least one of the user's posture and activity; determining an element adjustment of the presentation of at least one element of virtual content based on the determined user adjustment; and generating, based on the determined element adjustment, a control signal for adjusting the presentation of the at least one element of virtual content.

Proposed concepts thus aim to provide schemes, solutions, concept, designs, methods and systems pertaining to controlling the presentation of virtual content provided to a user by a mixed reality system.

In particular, embodiments aim to obtain user posture and user activity information, and, by analysing such information, to determine whether at least one of the user's posture and activity needs adjusting, and if so, determining a user adjustment. During analysis of the user posture and user activity information, ergonomics data describing reference postures and activities is considered in order to determine a user adjustment. For example, a user's posture while sitting can be compared to ergonomic data describing a sitting posture that is known to reduce common injuries, and a user adjustment needed to bring the user in line with the 'correct' sitting posture may then be determined. After the user adjustment has been determined, an element adjustment of the presentation of at least one element of virtual content can be determined based on the user adjustment. For example, if the user adjustment is to make the user stand up, the element adjustment may be to move an element of virtual content up, relative to the user, so that the user must stand to be able to see it. A control signal for implementing the element adjustment may then be generated in order to communicate with the mixed reality system or an application of the mixed reality system.

In other words, it is proposed that obtained user posture and user activity information may be analysed to determine a user adjustment of a user's posture and/or activity. An element adjustment of the presentation of at least one element of virtual content can then be determined based on the user adjustment in such a way that it induces the determined user adjustment in the user. That is, for example, an element adjustment may be determined in order to help the user maintain a good working posture in a non-invasive way. This could be done, for example, by tilting an element of virtual content in such a way that the user must tilt their head to look at the element properly.

By providing a computer-implemented method of controlling the presentation of virtual content, adjustments of the user's posture and/or activity may be non-invasively induced. This may be to guide the user to sit properly, to stand, to walk, and more generally to act and stand/sit in a way that may help to prevent/reduce injuries or muscle strain. Embodiments may therefore have wide application in the field of mixed reality devices, including augmented reality devices and virtual reality devices, and for a wide range of activities using these devices, for instance working in a virtual office, gaming, or using a virtual screen.

By generating a control signal, embodiments may support interacting with a wide variety of applications of a mixed reality system, so that the current invention may not be limited to aiding the user only when the mixed reality system is being used in a specific way.

For instance, by automatically obtaining user posture and user activity information, determining a user adjustment based on the obtained user posture and activity information, and based on ergonomics data, determining an element adjustment of the presentation of at least one element of virtual content based on the determined user adjustment, a control signal for adjusting the presentation of an element of virtual content, corresponding to the determined element adjustment, may be generated.

The use of steering elements of virtual content in such a way as to induce an adjustment of a user's posture and/or activity provides a specific non-invasive way to guide the user to act in a way that may help them in avoiding common injuries associated with using a mixed reality system.

Ultimately, a way to actively adjust the presentation of virtual content in order to guide a user of a mixed reality system to act in a way that may aid them in avoiding common injuries that come along with using a mixed reality system may be supported by the proposed concept(s). Automatic and/or dynamic virtual content control concepts may thus be provided proposed embodiments.

In some embodiments, the ergonomics data comprises information describing reference postures and activities, predetermined guidance on durations of the reference postures and activities, posture dynamics, and/or muscle-skeleton models. This may include guidance on best practices to avoid RSI, or neck, arm and back strains. For instance, the ergonomic data may comprise information on how long it is healthy to stay sitting for, or the best posture for sitting, or, for instance, how long to stand in one place, or how straight the user's neck should be in order to avoid neck injuries.

In some embodiments, determining a user adjustment comprises selecting a reference posture and/or activity from the ergonomics data. This allows the determining of a new activity or posture that may aid the user in avoiding injury or muscle strain, dependent on their current posture and/or activity. For instance, if the user's current activity is sitting, the reference activity selected may be standing. In another example, if the user is standing but significantly leaning to the right, the reference activity selected may be standing straight.

In some embodiments, determining a user adjustment further comprises comparing user posture and user activity information with the selected reference posture and/or activity. This may allow the specific motions needed to bring a user from their current posture and/or activity into the desired posture and/or activity to be determined. For instance, in the example of a user currently sitting, and the selected reference activity being standing, comparing the posture of the user sitting with the posture of a reference user standing allows the needed user adjustment to be determined, the user adjustment in this case being the user standing up. Or, for example, if the user's neck is currently too far forward, and a reference posture has the user's neck straighter, the difference in angle between the user's current neck position and the selected reference posture can be determined and utilized in determining the required user adjustment.

In some embodiments, the user adjustment is to make the user: stand up, walk in a particular direction, walk in a circle, sit with a straight back, or look left/right/up/down. These user adjustments may be useful in common situations such as the user sitting for too long, not sitting properly, being stationary for too long, or straining their neck.

In some embodiments, the element adjustment is to: tilt the at least one element of virtual content, move the at least one element of virtual content, shrink the at least one element of virtual content, or stretch the at least one element of virtual content. These element adjustments may be useful in inducing the determined user adjustments in the user. For instance if the user is straining their neck, an element of virtual content may be tilted to steer the user to change their posture in such a way that they will no longer strain their neck. Or, if the user has been sitting for too long, an element of virtual content, for instance a virtual screen, may be moved upwards relative to the user so that the user must stand to be able to continue to see the screen. In another example, an element of virtual content may be shrunk unless the user is walking towards it in order to induce the user to walk in a desired direction.

In some embodiments, the method further comprises: obtaining user characteristics describing physical parameters of the user, and determining, further based at least partly on the obtained user characteristics, a user adjustment of at least one of the user's posture or activity. User characteristics, such as a user's height, weight, body shape, general health, possible physical limitations, and other relevant physical parameters of the user may have an impact on what type of postures and/or activities are healthy or possible for a particular user. User characteristics may also comprise medical background information of the user such as any chronic conditions. User characteristics may also include activity information for the user, for instance, information describing their fitness routines or workout activities.

In some embodiments, the method further comprises: obtaining information describing a current real-world environment of the user, and determining, further based at least partly on the obtained information describing a current real-world environment of the user, a user adjustment of at least one of the user's posture or activity. Information about the current environment of the user may have an impact on what types of postures and/or activities are possible.

In some embodiments, the method further comprises obtaining user posture and user activity information, describing posture and activity of at least a second user respectively, and determining, by further analyzing the obtained user posture and user activity information of the at least second user, a user adjustment of at least one of the at least two users' posture and activity. This may allow a system according to the present application to be used in a shared virtual environment, for instance in a virtual meeting room, taking into account the needs of multiple users. For instance, if a majority of users seem not to be engaged, i.e. leaning back in their chairs, a user adjustment to make the users stand up may be determined in order to keep the users engaged.

In some embodiments, obtaining user posture and user activity information comprises receiving the information from an external system. An external system may be the mixed reality system, for example, which typically already includes a gyroscope and an accelerometer and/or cameras that may be leveraged in order to obtain user posture and user activity information. Or, for instance, an external system may be a wearable fitness tracker that monitors the user's activity, a pair of controllers that track the locations of the user's hands, or an external camera with a view of the user.

In some embodiments, the method further comprises detecting user posture and user activity via at least one sensor, and generating user posture and user activity information based on the detected user posture and user activity. A sensor integrated into a system according to an embodiment of the present invention may allow user posture and user activity information to be obtained directly by the system and user posture and user activity information homogenized.

In some embodiments, the method further comprises obtaining application information from an application of the mixed reality system describing possible element adjustments, and determining an element adjustment further based on the obtained application information. Information describing an application of the mixed reality system may have an impact on what types of element adjustments are possible.

There is also provided a computer program product comprising computer program code means which, when executed on a computing device having a processing system, cause the processing system to perform all the steps of any herein disclosed method.

There is also provided a control system for controlling the presentation of virtual content in a virtual environment, provided to a user by a mixed reality system, the system being configured to: obtain user posture and user activity information describing posture and activity of the user respectively; determine, by analyzing the obtained user posture and user activity information, and based at least partly on ergonomics data describing reference postures and activities, a user adjustment of at least one of the user's posture and activity; determine an element adjustment of the presentation of at least one element of virtual content based on the determined user adjustment; and generate, based on the determined element adjustment, a control signal for adjusting the presentation of the at least one element of virtual content.

There is also provided a mixed reality system comprising the control system disclosed above.

Thus, there may be proposed concepts for controlling the presentation of virtual content, provided to a user by a mixed reality system.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a simplified flow diagram of a method for controlling the presentation of virtual content;
Fig. 2 is a more in-depth flow diagram of a method for controlling the presentation of virtual content according to a proposed embodiment;
Fig. 3 is a simplified flow diagram of a method for controlling the presentation of virtual content according to a proposed embodiment;
Fig. 4 is a simplified block diagram of a control system for controlling the presentation of virtual content according a proposed embodiment; and
Fig. 5 illustrates an example of a computer within which one or more parts of an embodiment may be employed.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

Implementations in accordance with the present disclosure relate to various techniques, methods, schemes and/or solutions pertaining to controlling the presentation of virtual content. According to proposed concepts, a number of possible solutions may be implemented separately or jointly. That is, although these possible solutions may be described below separately, two or more of these possible solutions may be implemented in one combination or another.

Embodiments of the invention aim to obtain user posture and user activity information, and by analysing it, to determine whether at least one of the user's posture and activity needs adjusting, and if so, determining a user adjustment. During analysis of the user posture and user activity information, ergonomics data describing reference postures and activities is considered in order to determine a user adjustment. For example, the user's posture while sitting can be compared to ergonomic data describing a sitting posture known to reduce common injuries, and a user adjustment needed to bring the user in line with the 'correct' sitting posture can be determined. After the user adjustment has been determined, an element adjustment of the presentation of at least one element of virtual content can be determined based on the user adjustment. For example, if the user adjustment is to make the user stand up, the element adjustment may be to move an element of virtual content up, relative to the user, so that the user must stand to be able to see it. A control signal for implementing the element adjustment can then be generated in order to communicate with the mixed reality system or an application of the mixed reality system.

Proposed concepts thus aim to provide schemes, solutions, concept, designs, methods and systems pertaining to controlling the presentation of virtual content, provided to a user by a mixed reality system.

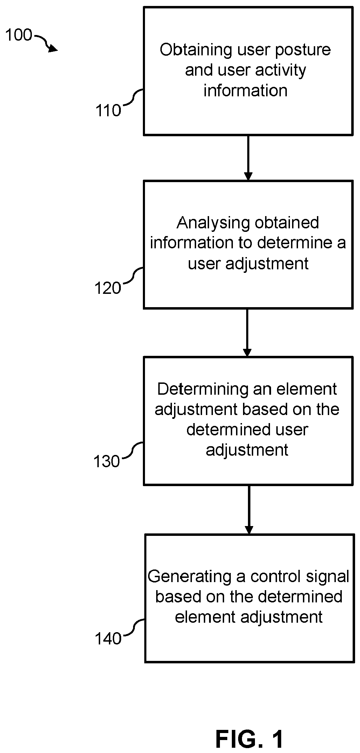

Referring now to Fig. 1, there is depicted a simplified flow diagram of a method 100 for controlling the presentation of virtual content, according to a proposed embodiment.

The method begins with the step 110 of obtaining user posture and user activity information describing posture and activity of the user respectively. Such information may be obtained from an external system. An external system may be the mixed reality system, for example, which typically already includes a gyroscope and an accelerometer and/or cameras that may be leveraged in order to obtain user posture and user activity information. Or, for instance, an external system may be a wearable fitness tracker that monitors the user's activity, a pair of controllers that monitor the locations of the user's hands, or an external camera with a view of the user.

In some embodiments, user posture and user activity information may comprise information describing a user talking. This information may be useful as a strain on the vocal cords may indicate the user is in a sub-optimal posture.

In step 120, the obtained user posture and user activity information is analyzed to determine, also based at least partly on ergonomics data describing reference postures and activities, a user adjustment of at least one of the user's posture and activity.

Ergonomics data may comprise information describing reference postures and activities, predetermined guidance on durations of the reference postures and activities, posture dynamics, and/or muscle-skeleton models. This may include guidance on best practices to avoid RSI, or neck, arm and back strains. For instance, the ergonomic data may comprise information on how long it is healthy to stay sitting for, or the best posture for sitting, or, for instance, how long to stand in one place, or how straight the user's neck should be in order to avoid neck injuries.

Determining a user adjustment may comprise selecting a reference posture and/or activity from the ergonomics data. This allows the determining of a new activity or posture that may aid the user in avoiding injury or muscle strain, dependent on their current posture and/or activity. For instance, if the user's current activity is sitting, the reference activity selected may be standing. In another example, if the user is standing but significantly leaning to the right, the reference activity selected may be standing straight. In some embodiments, determining a user adjustment may further comprise comparing user posture and user activity information with the selected reference posture and/or activity. This may allow the specific motions needed to bring a user from their current posture and/or activity into the desired posture and/or activity to be determined. For instance, in the example of a user currently sitting, and the selected reference activity being standing, comparing the posture of the user sitting with the posture of a reference user standing allows the needed user adjustment to be determined, the user adjustment in this case being the user standing up. Or for example, if the user's neck is currently too far forward, and a reference posture has the user's neck straighter, the difference in angle between the user's current neck position and the selected reference posture can be determined and utilized in determining the required user adjustment.

Another example of how user activity information may be analyzed to determine a user adjustment is, for instance, considering the nature of the activity the user is performing. For example, if the user is in a virtual meeting, a break in the meeting may be waited for until determining a user adjustment of making the user stand. Or, for example, if the user is engaged in a particular work task that involves high focus, the determined user adjustment may be such as to minimize interruption to the user's focus.

In some embodiments, the user adjustment may be to make the user: stand up, walk in a particular direction, walk in a circle, sit with a straight back, or look left/right/up/down. These user adjustments may be useful in common situations such as the user sitting for too long, not sitting properly, being stationary for too long, or straining their neck.

In step 130, an element adjustment of the presentation of at least one element of virtual content is determined based on the determined user adjustment. An element of virtual content may be, for example, a virtual screen, an avatar, or an interactive object.

In some embodiments, the element adjustment may be to: tilt the at least one element of virtual content, move the at least one element of virtual content, shrink the at least one element of virtual content, or stretch the at least one element of virtual content. These element adjustments may be useful in inducing the determined user adjustments in the user. For instance if the user is straining their neck, an element of virtual content may be tilted to steer the user to change their posture in such a way that they will no longer strain their neck. Or, if the user has been sitting for too long, an element of virtual content, for instance a virtual screen, may be moved upwards relative to the user so that the user must stand to be able to continue to see the screen. An element of virtual content may also be shrunk unless the user is walking towards it in order to induce the user to walk in a desired direction. An element of virtual content may also be moved around in a circle to make the user walk in a circle.

Information regarding human cognition and the nature of human perception may be used in order to aid in determining the type of element adjustment needed to induce the determined user adjustment.

In step 140, a control signal for adjusting the presentation of the at least one element of virtual content is generated, based on the determined element adjustment. By generating a control signal, embodiments may support interacting with a wide variety of applications of a mixed reality system, so that the current invention may not be limited to aiding the user only when the mixed reality system is being used in a specific way or only using one application. Applications of a mixed reality system may have interfaces to interact with a system according to any herein described embodiment.

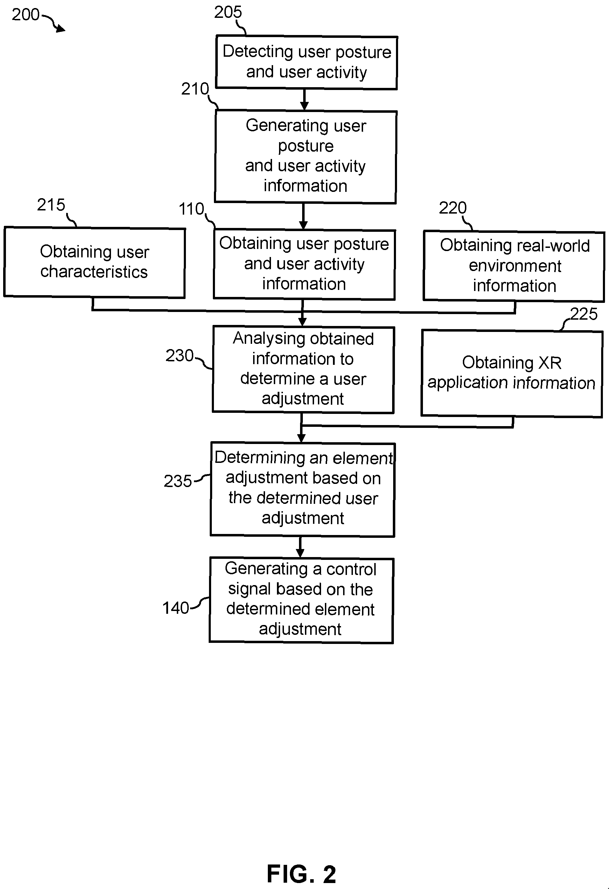

Referring now to Fig. 2, there is provided a more in-depth flow diagram of a method 200 for controlling the presentation of virtual content, according to a proposed embodiment.

In step 205, user posture and user activity is detected via at least one sensor. A sensor integrated into a system according to an embodiment of the present invention may allow user posture and user activity information to be obtained directly by the system.

In step 210, user posture and user activity information may be generated based on the detected user posture and user activity. Generating the user posture and user activity information from user posture and user activity detected directly by a system according to an embodiment of the present invention may allow the user posture and user activity information to be more homogenized across various applications of the system, which may streamline the analysis of the obtained information.

In step 110, user posture and user activity information is obtained, as described above regarding method 100.

In step 215, user characteristics describing physical parameters of the user are obtained, upon which the determining of a user adjustment in step 230 may be partly based. User characteristics, such as a user's height, weight, body shape, general health, possible physical limitations, and other relevant physical parameters of the user may have an impact on what type of postures and/or activities are healthy or possible for a particular user. User characteristics may also comprise medical background information of the user such as any chronic conditions. User characteristics may also include activity information for the user, for instance, information describing their fitness routines or workout activities.

In step 220, information describing a current real-world environment of the user is obtained, upon which the determining of a user adjustment in step 230 may be partly based. Information about the current environment of the user may have an impact on what types of postures and/or activities are possible. This information may be obtained via the mixed reality system, via embedded cameras, other sensors, or safety fence information stored in the mixed reality system or set by a user of the mixed reality system.

The real-world environment of the user comprises, for example, objects around the user, the dimensions of the room they are in, and other people or animals around the user. It may also comprise the lighting and temperature of the room the user is in, and the nature of any audio around the user. This information may be obtained via a connected Internet of Things system, to which a system according to any herein described embodiment may be connected.

In step 230, the user posture and user activity information obtained in step 110 is analyzed to determine a user adjustment of at least one of the user's posture and activity. The determination is further based on ergonomics data describing reference postures and activities, user characteristics obtained in step 215, and information describing a real-world environment of the user obtained in step 220.

In step 225, application information from an application of the mixed reality system is obtained describing possible element adjustments. Information describing an application of the mixed reality system may have an impact on what types of element adjustments are possible in the application the user is currently using.

In step 235, an element adjustment of the presentation of at least one element of virtual content is determined based on the user adjustment determined in step 230 and further based on the application information obtained in step 225.

In step 140, a control signal for adjusting the presentation of the at least one element of virtual content is generated, based on the determined element adjustment, as described above in regards to method 100.

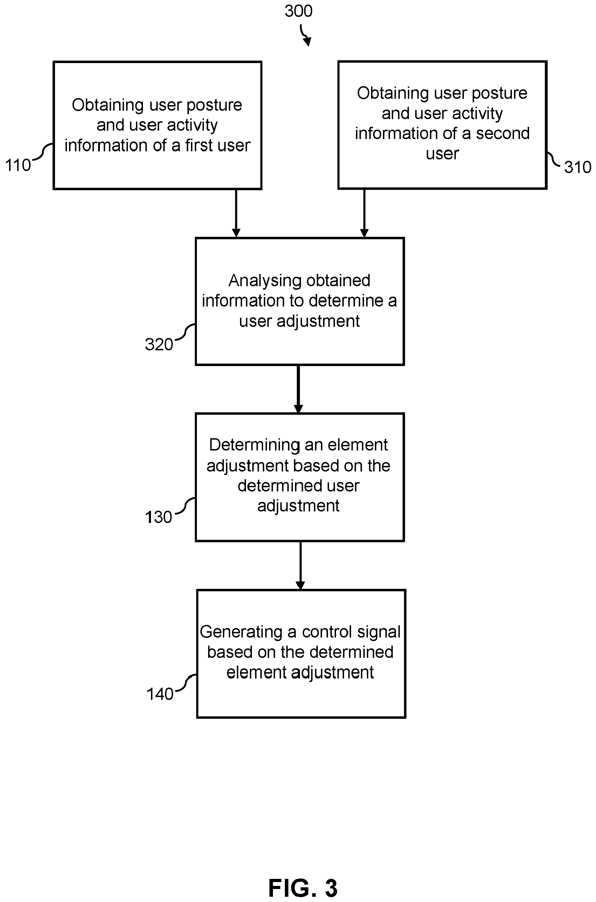

Referring now to Fig. 3, there is depicted a simplified flow diagram of a method 300 for controlling the presentation of virtual content, according to a proposed embodiment.

In step 110, user posture and user activity information describing posture and activity of a first user respectively is obtained.

In step 310, user posture and user activity describing posture and activity of a second user respectively is obtained.

In step 320, the obtained user posture and user activity information of both users is analysed to determine a user adjustment of at least one of both users' posture and activity. This may allow a system according to an embodiment of the present application to be used in a shared virtual environment, for instance a virtual meeting room, taking into account the needs of multiple users. For instance, if a majority of users seem not to be engaged, i.e. leaning back in their chairs, a user adjustment to make the users stand up may be determined in order to keep the users engaged.

Steps 130 and 140 are performed as already described in regards to method 100.

Method 300 may be used as a tool for moving groups of people around in a shared virtual environment, either presented to the users as augmented or virtual reality. For instance, the current embodiment could be used to keep the group engaged in an activity or to aid in preventing common injuries amongst the users. Method 300 is contrasted against method 100 in virtue of obtaining and analyzing user posture and user activity information describing the posture and activity of at least two users. A user adjustment is then determined for adjusting at least one of the users' posture and activity in the same way. An element adjustment is then determined, based on the user adjustment, for inducing the user adjustment in at least the two users. This method may form a continuous feedback loop, in which once the users have adjusted according to the determined user adjustment, user posture and user activity information is obtained again.

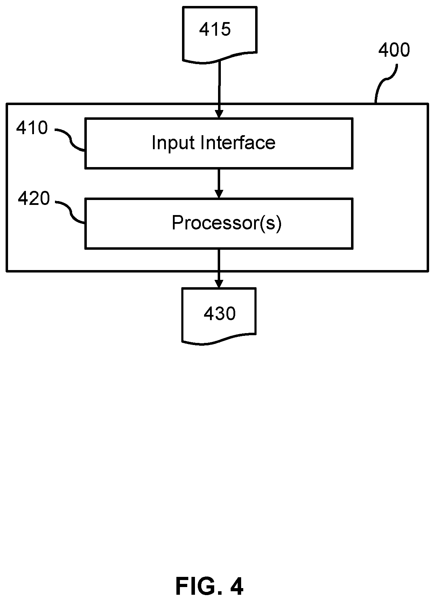

Referring now to Fig. 4, there is depicted a simplified block diagram of a control system 400 for controlling the presentation of virtual content, presented to a user by a mixed reality system, according an embodiment. The control system configured for controlling the presentation of virtual content comprises an input interface 410, and one or more processors 420.

The control system 400 is configured to analyse user posture and user activity information describing posture and activity of the user respectively. User posture and user activity information 415 is obtained. The system outputs an output 430 comprising a control signal for adjusting the presentation of at least one element of virtual content. The output 430 is generated based on a determined element adjustment, which itself is based on a determined user adjustment, which itself has been determined based on the obtained user posture and user activity information, and at least partly on ergonomics data. The output 430 is for interacting with the mixed reality system or an application of the mixed reality system.

In more detail, the input interface 410 receives user posture and user activity information 415 from either an external system or from at least one sensor of the system (not shown in this embodiment). The input interface 410 provides the user posture and user activity information 415 to one or more processors 420 for analysis to determine a user adjustment of at least one of the user's posture and activity.

Based on the determined user adjustment, the processor(s) 420 then determines an element adjustment of the presentation of at least one element of virtual content. The element adjustment is then processed by the processor(s) 420 to produce an output 430 comprising a control signal for adjusting the presentation of the at least one element of virtual content.

The control system 400 may be in direct communication with a mixed reality system, or itself form part of a mixed reality system.

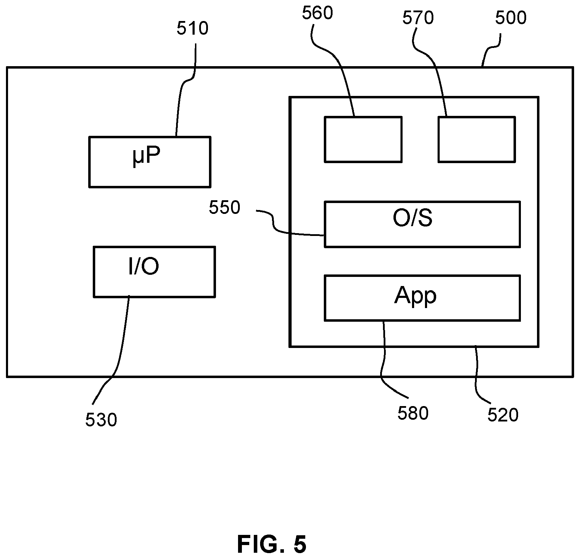

Fig. 5 illustrates an example of a computer 500 within which one or more parts of an embodiment may be employed. Various operations discussed above may utilize the capabilities of the computer 500. In this regard, it is to be understood that system functional blocks can run on a single computer or may be distributed over several computers and locations (e.g. connected via internet).

The computer 500 includes, but is not limited to, PCs, workstations, laptops, PDAs, palm devices, servers, storages, and the like. Generally, in terms of hardware architecture, the computer 500 may include one or more processors 510, memory 520 and one or more I/O devices 530 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 500, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 560, source code 570, and one or more applications 580 in accordance with exemplary embodiments. As illustrated, the application 580 comprises numerous functional components for implementing the features and operations of the exemplary embodiments. The application 580 of the computer 500 may represent various applications, computational units, logic, functional units, processes, operations, virtual entities, and/or modules in accordance with exemplary embodiments, but the application 580 is not meant to be a limitation.

The operating system 550 controls the execution of other computer programs, and provides scheduling, input-output control, file and data management, memory management, and communication control and related services. It is contemplated by the inventors that the application 580 for implementing exemplary embodiments may be applicable on all commercially available operating systems.

Application 580 may be a source program, executable program (object code), script, or any other entity comprising a set of instructions to be performed. When a source program, then the program is usually translated via a compiler (such as the compiler 560), assembler, interpreter, or the like, which may or may not be included within the memory 520, so as to operate properly in connection with the O/S 550. Furthermore, the application 580 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 530 may include input devices such as, for example but not limited to, a mouse, keyboard, scanner, microphone, camera, etc. Furthermore, the I/O devices 530 may also include output devices, for example but not limited to a printer, display, etc. Finally, the I/O devices 530 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 530 also include components for communicating over various networks, such as the Internet or intranet.

If the computer 500 is a PC, workstation, intelligent device or the like, the software in the memory 520 may further include a basic input output system (BIOS) (omitted for simplicity). The BIOS is a set of essential software routines that initialize and test hardware at start-up, start the O/S 550, and support the transfer of data among the hardware devices. The BIOS is stored in some type of read-only-memory, such as ROM, PROM, EPROM, EEPROM or the like, so that the BIOS can be executed when the computer 800 is activated.

When the computer 500 is in operation, the processor 510 is configured to execute software stored within the memory 520, to communicate data to and from the memory 520, and to generally control operations of the computer 500 pursuant to the software. The application 580 and the O/S 550 are read, in whole or in part, by the processor 510, perhaps buffered within the processor 510, and then executed.

When the application 580 is implemented in software it should be noted that the application 580 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 580 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The methods of Figs. 1-3, and the system of Fig. 4, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out an encoding or decoding method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, the blocks shown in the block diagrams of Fig. 4 may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A computer-implemented method for controlling the presentation of virtual content, provided to a user by a mixed reality system, the computer-implemented method comprising:
obtaining user posture and user activity information describing posture and activity of the user respectively;
determining, by analyzing the obtained user posture and user activity information, and based at least partly on ergonomics data describing reference postures and activities, a user adjustment of at least one of the user's posture and activity;
determining an element adjustment of the presentation of at least one element of virtual content based on the determined user adjustment; and
generating, based on the determined element adjustment, a control signal for adjusting the presentation of the at least one element of virtual content.

2. The method of claim 1, wherein ergonomics data comprises information describing reference postures and activities, predetermined guidance on durations of the reference postures and activities, posture dynamics, and/or muscle-skeleton models.

3. The method of claim 1 or 2, wherein determining a user adjustment comprises selecting a reference posture and/or activity from the ergonomics data.

4. The method of claim 3, wherein determining a user adjustment further comprises comparing user posture and user activity information with the selected reference posture and/or activity.

5. The method of any of claims 1 to 4, wherein the user adjustment is to make the user: stand up, walk in a particular direction, walk in a circle, sit with a straight back, or look left/right/up/down.

6. The method of any of claims 1 to 5, wherein the element adjustment is to: tilt the at least one element of virtual content, move the at least one element of virtual content, shrink the at least one element of virtual content, or stretch the at least one element of virtual content.

7. The method of any of claims 1 to 6, wherein the method further comprises:
obtaining user characteristics describing physical parameters of the user; and
determining, further based at least partly on the obtained user characteristics, a user adjustment of at least one of the user's posture or activity.

8. The method of any of claims 1 to 7, wherein the method further comprises:
obtaining information describing a current real-world environment of the user; and
determining, further based at least partly on the obtained information describing a current real-world environment of the user, a user adjustment of at least one of the user's posture or activity.

9. The method of any of claims 1 to 8, wherein the method further comprises:
obtaining user posture and user activity information, describing posture and activity of at least a second user respectively; and
determining, by further analyzing the obtained user posture and user activity information of the at least second user, a user adjustment of at least one of the at least two users' posture and activity.

10. The method of any of claims 1 to 9, wherein obtaining user posture and user activity information comprises receiving the information from an external system.

11. The method of any of claims 1 to 10, wherein the method further comprises:
detecting user posture and user activity via at least one sensor; and
generating user posture and user activity information based on the detected user posture and user activity.

12. The method of any of claims 1 to 11, wherein the method further comprises:
obtaining application information from an application of the mixed reality system describing possible element adjustments;
determining an element adjustment further based on the obtained application information.

13. A computer program product comprising computer program code means which, when executed on a computing device having a processing system, cause the processing system to perform all the steps of the method according to any of claims 1 to 12.

14. A control system for controlling the presentation of virtual content in a virtual environment, provided to a user by a mixed reality system, the system being configured to:
obtain user posture and user activity information describing posture and activity of the user respectively;
determine, by analyzing the obtained user posture and user activity information, and based at least partly on ergonomics data describing reference postures and activities, a user adjustment of at least one of the user's posture and activity;
determine an element adjustment of the presentation of at least one element of virtual content based on the determined user adjustment; and
generate, based on the determined element adjustment, a control signal for adjusting the presentation of the at least one element of virtual content.

15. A mixed reality system comprising the control system of claim 14.
